# EUROPEAN PATENT APPLICATION

(11) **EP 2 992 882 A1**
(43) Date of publication of application: **09.03.2016**
(21) Application number: 15183975.0
(22) Date of filing: 04.09.2015
(51) Int. Cl.: A61K 31/4172, A61P 27/12, A61P 35/00, A61P 17/18, A61Q 17/00

(54) **USE OF ERGOTHIONEINE FOR INDUCING ACTIVITY OF NRF2 IN CELL**

(30) Priority: 05.09.2014 TW 103130810
(71) Applicant: China Medical University, Taichung City 404 (TW)
(72) Inventor: HSEU, You-Cheng, Taichung City (TW); YANG, Hsin-Ling, Taichung City (TW)
(74) Representative: Lang, Christian

(57) **Abstract**

A use of an ergothioneine is disclosed. The use of the ergothioneine is manufacture of a product for inducing an activity of an Nrf2 in a cell, wherein the cell is a normal cell or a damaged cell resulting from an exposure to an ultraviolet radiation (UVR).

## Description

### BACKGROUND

### Technical Field

The present disclosure relates to a novel use of an ergothioneine. More particularly, the present disclosure relates to the novel use of the ergothioneine for inducing an activity of an Nrf2 in a cell.

### Description of Related Art

An irradiation of an ultraviolet radiation (UVR) from a sun is one of oxidative stresses that human contact most frequently in life. An overexposure of the UVR can cause serious illnesses such as a cataract and a skin cancer. Although tolerances of UVR in different races are different, a bodily harm will be caused by accumulated amount of a sunlight to a limit.

A skin, the largest organ in a body, is an important barrier of the body to isolate external damages. The overexposure of the UVR is one of main reasons of an environment-induced skin damage, and it causes an erythema or a sunburn. A long-term exposure to the UVR can result in a skin photoaging, which generates wrinkles, an anetoderma and the skin cancer. The UVR is invisible, but damages caused by the UVR can not be ignored. Therefore, preventions and treatments of UVR-induced damages are very important.

The irradiation of the UVR will generate free radicals. It will produce the oxidative stress when the free radicals produced by the irradiation of the UVR and an antioxidant system in the body can not maintain a constant state. Previously studies have demonstrated that antioxidants can scavenge the free radicals in the body, reduce the oxidative stress, and prevent and treat diseases. The daily contacted UVR can be subdivided into a number of ranges into an ultraviolet A (UVA) with a wavelength from 320 nm to 400 nm, an ultraviolet B (UVB) with the wavelength from 280 nm to 320 nm, and an ultraviolet C (UVC) with the wavelength from 100 nm to 280 nm. Most of the UVC, a shorter wave, is absorbed by an ozone layer in an atmosphere, and only a very small amount of the UVC reaches an Earth's surface. The wavelength of the UVA is the longest and energy of the UVA is the lowest. Although energy of the UVA is lower than the energy of the UVB, a penetration of the UVA is stronger than the penetration of the UVB. The UVA can penetrate into a dermis to cause acute and chronic damages, which tan the skin and degrade a collagen resulting in a skin aging and a wrinkle formation. Moreover, the UVA is the main risk factor for the skin cancer.

### SUMMARY

According to one aspect of the present disclosure, a use of an ergothioneine in manufacture of a product for inducing an activity of an Nrf2 in a cell.

In one example, the cell can be a normal cell or a damaged cell.

In one example, the damaged cell can result from an exposure to an ultraviolet radiation (UVR).

In one example, the UVR can be an ultraviolet A (UVA) with a wavelength from 320 nm to 400 nm.

In one example, the ergothioneine can be used in manufacture of the product for inhibiting an apoptosis caused by a stimulation of the UVA.

In one example, the ergothioneine can be used in manufacture of the product for increasing the expression of Nrf2-mediated genes expressing an enzymatic antioxidant.

In one example, the enzymatic antioxidant can be selected from the group consisting of heme oxygenase-1 (HO-1), NAD(P)H:quinone oxidoreductase 1 (NQO1), and glutamate-cysteine ligase catalytic subunit (GCLC).

In one example, the ergothioneine can be used in manufacture of the product for increasing the expression of the Nrf2-mediated genes expressing a non-enzymatic antioxidant.

In one example, the non-enzymatic antioxidant can be a glutathione (GSH).

In one example, the ergothioneine can be used in manufacture of the product for inducing a nuclear translocation of the Nrf2.

In one example, a therapeutically effective amount of the ergothioneine can range from 125 nM to 500 nM.

### BRIEF DESCRIPTION OF THE DRAWINGS

The present disclosure can be more fully understood by reading the following detailed description of the embodiment, with reference made to the accompanying drawings as follows:
Fig. 1 illustrates an effect of an ergothioneine on a cell viability of a UVA-irradiated human keratinocytes;
Fig. 2 illustrates the effect of the ergothioneine on a reactive oxygen species (ROS) generation of the UVA-irradiated human keratinocytes;
Fig. 3 illustrates the effect of the ergothioneine on a lactate dehydrogenase (LDH) release of the UVA-irradiated human keratinocytes;
Fig. 4 illustrates the effect of the ergothioneine on a DNA damage of the UVA-irradiated human keratinocytes;
Fig. 5 illustrates the effect of the ergothioneine on an apoptosis of the UVA-irradiated human keratinocytes;
Fig. 6 illustrates the effect of the ergothioneine on protein expressions of apoptosis-related proteins of the UVA-irradiated human keratinocytes;
Fig. 7 illustrates the effect of the ergothioneine on the expressions of a Nrf2 signaling pathway associated proteins of human keratinocytes;
Fig. 8 illustrates the effect of the ergothioneine on the expressions of the Nrf2 signaling pathway associated proteins of the UVA-irradiated human keratinocytes;
Fig. 9 illustrates the effect of the ergothioneine on the protein expressions of a cytosolic Nrf2 and a nuclear Nrf2 of the UVA-irradiated human kerati nocytes;
Fig. 10 illustrates the effect of the ergothioneine on a content of a glutathione (GSH) of the UVA-irradiated human keratinocytes;
Fig. 11 illustrates the effect of the ergothioneine on a transcriptional activity of an antioxidant response element (ARE);
Fig. 12 illustrates that the ergothioneine induces a Nrf2-mediated antioxidant signaling pathway;
Fig. 13-(A) illustrates the effect of the ergothioneine on protein expression of the Nrf2 in Nrf2 knockdown human keratinocytes by using a siRNA transfection;

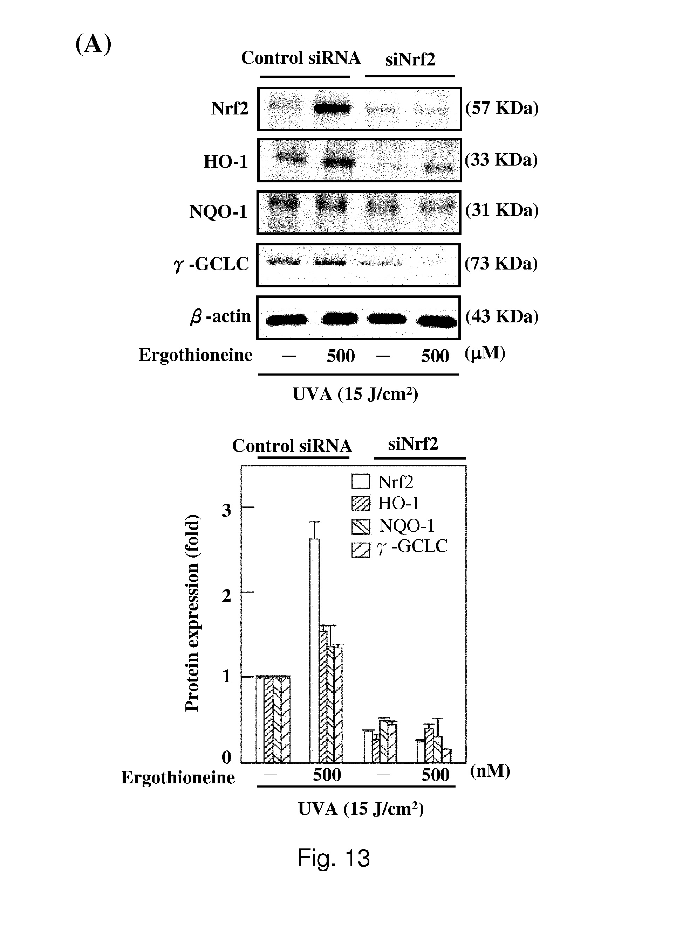

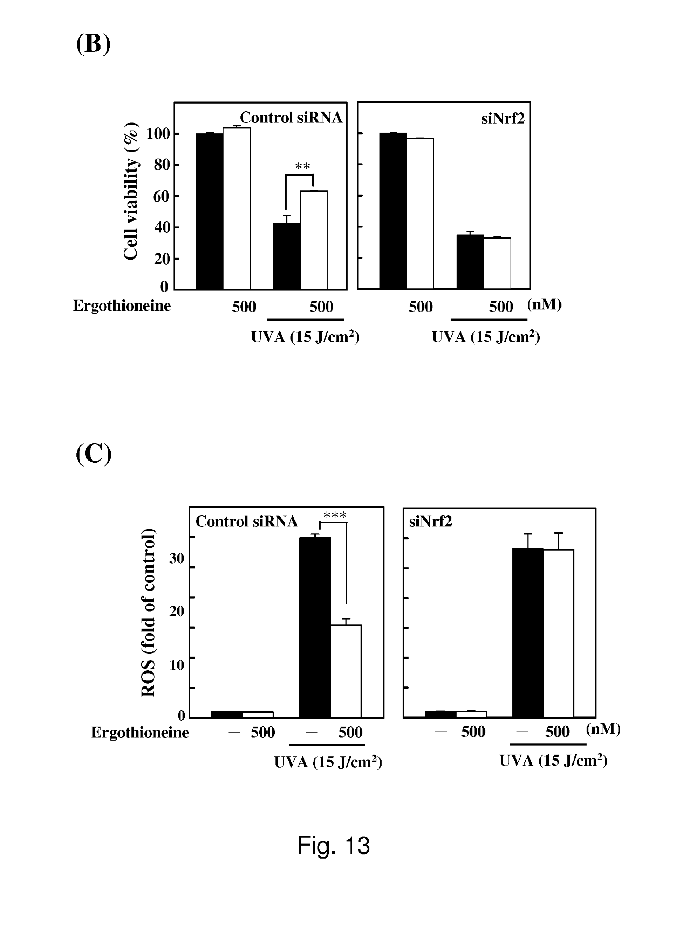

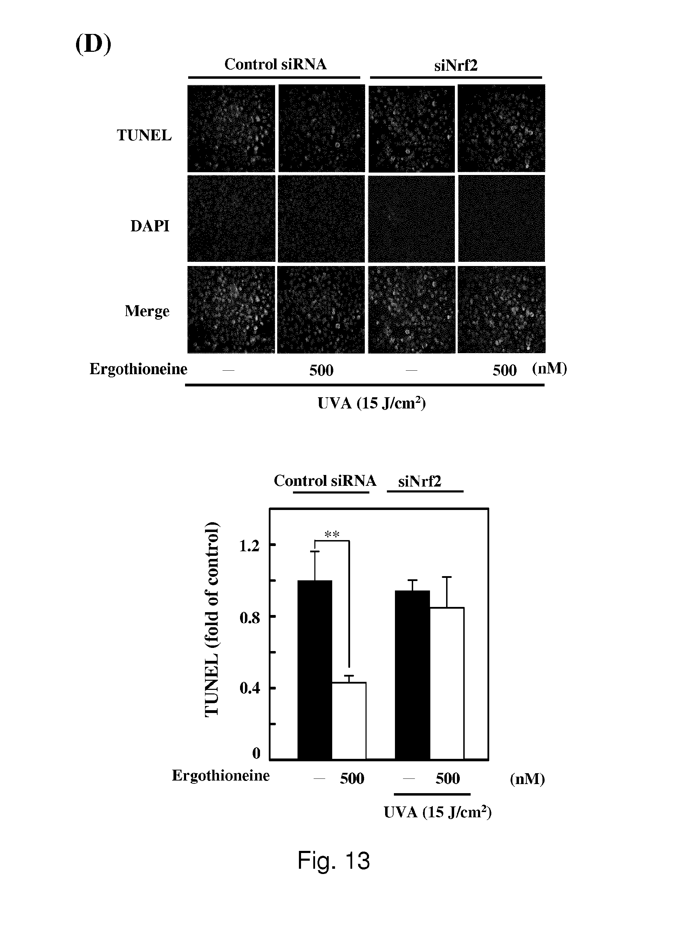

Fig. 13-(B) illustrates the effect of the ergothioneine on the cell viability in Nrf2 knockdown human keratinocytes by using the siRNA transfection;
Fig. 13-(C) illustrates the effect of the ergothioneine on a ROS generation of the UVA-irradiated human keratinocytes by using the siRNA transfection; and
Fig. 13-(D) illustrates the effect of the ergothioneine on the apoptosis of the UVA-irradiated human keratinocytes by using the siRNA transfection.

### DETAILED DESCRIPTION

A novel use of an ergothioneine is provided for inducing an activity of an Nrf2 in a cell. According to results of *in vitro* human keratinocyte experiment models, aforementioned ergothioneine is capable of increasing anti-photooxidation ability of human keratinocytes through inducing the activity of the Nrf2. Furthermore, the ergothioneine is capable of inducing a nuclear translocation of the Nrf2 to enhance an expression of downstream antioxidant genes, and inhibiting an ultraviolet A (UVA)-induced apoptosis.

The term "ergothioneine" refers to 2-mercapto histidine trimethylbetain. The ergothioneine, a rare amino acid, is only formed in certain bacteria and fungi. A human body can not synthesize the ergothioneine, hence the ergothioneine is only taken from a food supply, mainly through an edible mushroom intake. Previously studies have demonstrated that the ergothioneine has radiation protective properties in capturing singlet oxygen, hydroxyl radicals and lipid peroxidation free radicals, an anti-inflammatory property, an anti-mutagenic property and a neuroprotective property. However, the ergothioneine is needed to use a higher concentration such as 0.5 mM in order to achieve its effectiveness in previous studies.

The term "Nrf2 (nuclear factor erythroid 2-related factor 2)" refers to a redox-sensitive transcription factor that binds to an antioxidant response element (ARE), a cis-acting DNA regulatory element of antioxidant enzyme genes, to promote gene expressions of downstream antioxidant enzymes. Under normal physiological condition, the Nrf2 binds to Kelch-like ECH-associated protein 1 (Keap-1) in a cytoplasm, and then the Nrf2 is degraded through ubiquitinated proteasomal degradation. When the Nrf2 is activated, a Nrf2-keap1 complex is degraded so that the Nrf2 translocates into a nucleus and recruits small Maf (sMaf) protein. A Nrf2-sMaf heterodimer then binds to the ARE that induce a transcription activity of a promoter region to promote the gene expressions of the downstream antioxidant enzymes.

Reference will now be made in detail to the present embodiments of the present disclosure, examples of which are illustrated in the accompanying drawings. Wherever possible, the same reference numbers are used in the drawings and the description to refer to the same or like parts.

### Examples

### I. Effects of the ergothioneine on anti-photooxidation ability of human keratinocytes

### 1.1 The effect of the ergothioneine and the UVA on a cytotoxicity of the human keratinocytes

To examine a safety of the ergothioneine and a safe used dose of the ergothioneine to normal cells, HaCaT cells, human keratinocyte cell lines, are treated with different dose of the ergothioneine and then detected a cell viability.

The HaCaT cells are maintained at 37°C in a humidified atmosphere of 5% CO₂ in a culture medium (Dulbecco's modified Eagle medium/high glucose (DMEM) supplemented with 10% heat-inactivated fetal bovine serum (FBS)).

The cell viability is monitored using a method of transcriptional and translational assay (MTT assay). The HaCaT cells are seeded in a 12-well plate with 1×10⁵ cells/well and then maintained at 37°C in a humidified atmosphere of 5% CO₂ untill the HaCaT cells are adhered in the next day. The HaCaT cells are subsequently incubated with or without the ergothioneine, and then maintained at 37°C in a humidified atmosphere of 5% CO₂ for 24 hours, wherein the concentration of the ergothioneine is 125 nM, 250 nM, and 500 nM, respectively. Next, the HaCaT cells are washed with phosphate buffered saline (PBS) three times, and then irradiated with the UVA (15 J/cm²). After the UVA irradiation, the PBS is replaced with the culture medium to culture the HaCaT cells for 4 hours. In next step, the culture medium is removed, the HaCaT cells are washed with PBS thrice, and then 450 µL of 1 × MTT agent is added in each well to react at 37°C in a humidified atmosphere of 5% CO₂ for 2 hours. A culture supernatant is removed and re-suspended with 400 µL of isopropanol to dissolve MTT formazan. 200 µL of the supernatant is taken from the well and then added into a 96-well microplate to measure an absorbance at 570 nm.

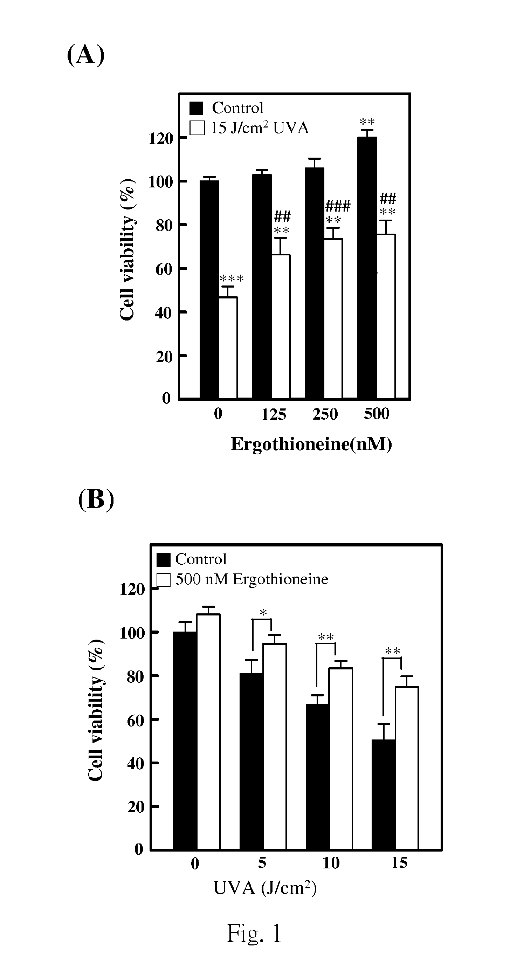

Fig. 1 illustrates the effect of the ergothioneine on the cell viability of UVA-irradiated HaCaT cells. Fig. 1-(A) is a quantitative diagram of the cell viability. A data result is represented by mean ± SD values; n = 3, wherein * represents p<0.05 compared to the control group, and # represents p<0.05 compared to the group exposed to the UVA. In Fig. 1-(A), the HaCaT cells are pretreated with the ergothioneine at the dose ranging from 125 nM to 500 nM, and then treated with or without the UVA irradiation. The low dose of the ergothioneine (125-500 nM) does not cause any cytotoxic effects. The cell viability of the group treated with the UVA irradiation drops to 46.6 %. However, the ergothioneine pretreatment attenuates a UVA-induced cell death in a dose-dependent manner. The cell viability of the gruop pretreated with 500 nM ergothioneine reverts to 75.5%.

To examine the effects of different dose of the UVA on the cell viability of the HaCaT cells, the HaCaT cells are treated with 500 nM ergothioneine, the highest non-cytotoxic concentration, for 2 hours, and then exposed to the UVA at the dose of 5 J/cm², 10 J/cm² and 15 J/cm², respectively. The cell viability of the HaCaT cells is detected by the MTT assay.

Fig. 1-(B) is a quantitative diagram of the cell viability. The data result is represented by mean ± SD values, n = 3, wherein * represents p<0.05 (the group treated with the ergothioneine compares to the group untreated with the ergothioneine at the same UVA dose). In the group untreated with the ergothioneine, the cell viability is decreased by the increased dose of the UVA irradiation, and the prominent cell death is observed at 15 J/cm² (IC₅₀=15.1). Accordingly, the dose of the UVA irradiation is 15 J/cm² used in the subsequent experiments. However, the increased cell death on respective dose of the UVA irradiation (5-15 J/cm²) is significantly attenuated by pretreating 500 nM ergothioneine.

### 1.2 The effect of the ergothioneine on a content of reactive oxygen species (ROS) induced by the UVA stimulation

An exposure of the UVA will stimulate cells generating ROS. To examine whether the ergothioneine is capable of inhibiting the ROS generation, or the ergothioneine per se contributes the ROS generation, an intracellular accumulation of the ROS is detected by a fluorescence microscopy and an enzyme-linked immunosorbent assay (ELISA) using a 2',7'-dichlorofluorescein diacetate (HDCF-DA).

The HDCF-DA is a lipid-soluble fluorescent dye and has a cell membrane permeability. The HDCF-DA will bind to intracellular esterases to form a non-fluorescent 2',7'-dichlorofluorescin (DCFH). The DCFH is subsequently oxidated by H₂O₂ to form a fluorescent 2',7'-dichlorofluorescein (DCF), and then accumulated in a mitochondria. Therefore, an emitting fluorescence of the DCF can represent an intracellular concentration of H₂O₂.

The HaCaT cells are seeded in a 12-well plate with 1×10⁵ cells/well and then maintained at 37°C in the humidified atmosphere of 5% CO₂ untill the HaCaT cells are reached 80% confluence. The HaCaT cells are subsequently incubated with or without the ergothioneine, and then maintained at 37°C in a humidified atmosphere of 5% CO₂ for 24 hours, wherein the concentration of the ergothioneine is 500 nM. Next, the HaCaT cells are washed with PBS three times, and then irradiated with the UVA (15 J/cm²). After the UVA irradiation, the PBS is replaced with 1 mL of HDCF-DA (10 µM) in each well to react at 37°C in the dark for 30 minutes. After the reaction, the fluorescent dye is removed, and the HaCaT cells are washed with PBS thrice to remove the remaining fluorescent dye. The intracellular ROS, as indicated by DCF fluorescence, is measured with the fluorescence microscope and photographed. After the fluorescence microscope observation, 500 µL of Triton-100 (0.25%) is added in each well for disrupting the cell membrane to release green fluorescence. 200 µl of the cell lysis is taken from the well, and then added into a 96-well black microplate to measure the absorbance by using a fluorescence spectrometer.

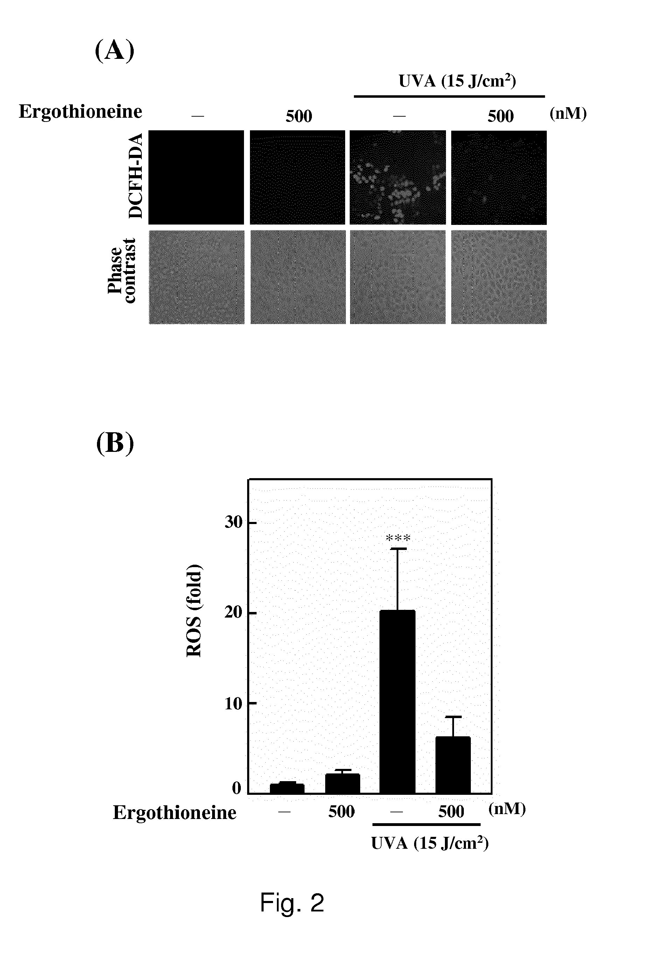

Fig. 2 illustrates the effect of the ergothioneine on the ROS generation of the UVA-irradiated HaCaT cells. Fig. 2-(A) shows intracellular ROS levels indicated by the DCF fluorescence and a cell morphology change measured by the fluorescence microscopy (200 × magnification). Fig. 2-(B) is the quantitative diagram showing a percentage of fluorescence intensity of DCF-stained cells. The data result is represented by mean ± SD values; n = 3, wherein * represents p<0.05 compared to the control group. There is non-effect of the group treated with 500 nM ergothioneine alone on the ROS generation. The group exposed to the UVA alone shows significant increase of fluorescence compared to the control group, or the group treated with the ergothioneine but untreated with the UVA irradiation. However, the fluorescence of the group treated with the ergothioneine prior to the 15 J/cm² UVA irradiation is significantly decreased. It indicates that the UVA-induced ROS generation is inhibited by the ergothioneine pretreatment.

### 1.3 The effect of the ergothioneine on an UVA-induced cell membrane damage

Under normal physiological condition, a lactate dehydrogenase (LDH) is presented in the cytoplasm. The cell membranes are damaged or disrupted when the cells are attacked by excessive ROS. Accordingly, the intracellular LDH releases into the culture medium. Therefore, a detection amount of LDH release is an indicator of a cell membrane damaged condition.

The HaCaT cells are seeded in a 12-well plate with 1×10⁵ cells/well and then maintained at 37°C in the humidified atmosphere of 5% CO₂ untill the HaCaT cells are adhered in the next day. The HaCaT cells are subsequently incubated with or without the ergothioneine and then maintained at 37°C in a humidified atmosphere of 5% CO₂ for 24 hours, wherein the concentration of the ergothioneine is 125 nM, 250 nM and 500 nM, respectively. After incubation, the HaCaT cells are washed with PBS three times and resuspended in fresh phenol red-free DMEM supplemented with 10% FBS. Then, the HaCaT cells are exposed to the UVA (15 J/cm²) for 4 hours. The culture medium is taken from the well, and then centrifuged at 400 × g for 5 minutes by using a high speed centrifuge. After the centrifugation, 50 µL of the supernatant is taken and added into the 96-well microplate, and the 50 µL of a substrate mixture is added in each well to act at room temperature for 20 minutes in the dark. Then, 50 µL of a stopping solution (1 N HCl) is added in each well to stop the reaction, and then the absorbance at 490 nm is measured.

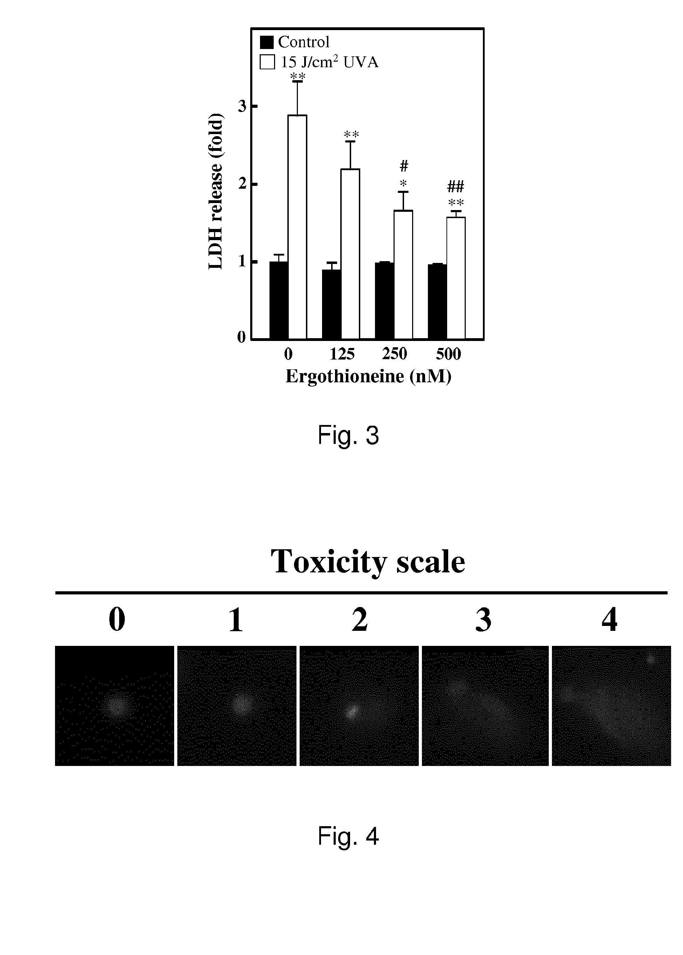

Fig. 3 illustrates the effect of the ergothioneine on the LDH release of the UVA-irradiated HaCaT cells, wherein Fig. 3 is the quantitative diagram showing a detection amount of the LDH release. The data result is represented by mean ± SD values; n = 3, wherein * represents p<0.05 compared to the control group. In Fig. 3, the exposure of the HaCaT cells to 15 J/cm² UVA enormously increases the LDH release into the culture medium, whereas the ergothioneine pretreatment inhibits the LDH release in a dose-dependent manner. These data further confirms that an UVA-induced membrane damage is significantly inhibited by the ergothioneine pretreatment.

### 1.4 The effect of the ergothioneine on an UVA-induced DNA damage

The exposure of the UVA can cause a DNA damage. To examine the effect of the ergothioneine on the UVA-induced DNA damage, we use a comet assay to analyze the DNA damage.

The comet assay involves an encapsulation of the cells in a low-melting-point agarose suspension, the cells lysis in alkaline (pH>13) conditions, a short-term electrophoresis of the suspended lysed cells, and a stained DNA of an electrophoretic stripe using the fluorescent dye. The term "comet" refers to a pattern of a DNA migration through the electrophoresis gel, wherein the DNA migration is caused by a DNA cleavage fragment of the damaged cell released from its nucleus, and the DNA cleavage fragment moves faster than the nucleus of a non-damaged cell in an electric field and results in structure resembling the comet.

The HaCaT cells are incubated with or without the ergothioneine for 24 hours, wherein the concentration of the ergothioneine is 125 nM, 250 nM and 500 nM, respectively. After incubation, the HaCaT cells are exposed to the UVA (15 J/cm²) for 4 hours. Then, the HaCaT cells are performed the comet assay and stained by red fluorescent dye. The pattern of the nucleus of the damaged cell resembles the comet in the comet assay, which is caused by the DNA cleavage fragment of the damaged cell. In contrast, the pattern of the nucleus of the normal cell, which has non-DNA cleavage fragment, is spherical in the comet assay.

Fig. 4 and Table 1 illustrate the effect of the ergothioneine on a DNA damage of the UVA-irradiated HaCaT cells. Fig. 4 is a fluorescent photomicrograph of the comet assay (200 × magnification). Table 1 shows a overall score of the comet assay. The data result is represented by mean ± SD values; n = 3, wherein * represents p<0.05 compared to the control group, and # represents p<0.05 compared to the group exposed to the UVA.

**Table 1**

| Group | Toxicity Scale | | | | | Total Damage |
|---|---|---|---|---|---|---|
| | 0 | 1 | 2 | 3 | 4 | |
| Control | 96±2 | 2±1 | 2±2 | 0±0 | 0±0 | 5±3 |
| 250 nM EGT | 88±4 | 10±4 | 2±2 | 0±0 | 0±0 | 14±4 |
| 500 nM EGT | 81±6 | 16±7 | 3±2 | 0±0 | 0±0 | 22±6 |
| 15 J/cm² UVA | 0±0 | 0±0 | 4±3 | 36±7 | 60±4 | 356±2*** |
| 250 nM EGT + 15 J/cm² UVA | 20±1 | 27±6 | 52±9 | 0±0 | 0±0 | ### 144±10*** |
| 500 nM EGT + 15 J/cm² UVA | 49±7 | 43±8 | 8±1 | 0±0 | 0±0 | ### 59±7*** |

According to the results of the fluorescent photomicrograph, comet-like DNA formations are categorized into five classes (0, 1, 2, 3, or 4) representing increasing DNA damage shown as a "tail". Table 1 shows the overall score for 100 comets ranged from 0 (100% of the comets in class 0) to 400 (100% of the comets in class 4). In Fig. 4 and Table 1, the UVA irradiation (15 J/cm²) for 4 hours triggers the DNA breakage of the nucleus shown as the tail. The overall score of the group treated with the UVA irradiation alone is 356.3 ± 2.1. However, the overall score of the group treated with the ergothioneine prior to the UVA irradiation drops to 59.0 ± 7.0. It indicates that the ergothioneine pretreatment can effectively reduce a tailing extent and a tailing number. Therefore, the ergothioneine can inhibit the DNA damage and has a protective property.

### 1.5 The effect of the ergothioneine on an UVA-induced apoptosis

The apoptosis can induce DNA double-strand breaks to generate a DNA fragment. To examine the effect of the ergothioneine on the UVA-induced apoptosis, an apoptotic cell death is measured using a terminal deoxyucleotidyl transferase-meditated dUTP-fluorescein nick end-labeking (TUNEL) assay, and protein expressions of apoptosis-related proteins, Caspase-3, Caspase-9, Bax and Bcl-2, are measured using a Western blot analysis. Therefore, the aforementioned methods in this example can analyze whether pretreating with the ergothioneine improves the UVA-induced apoptosis.

A chromosome breakage represents an initial stage of a cellular atrophy. The TUNEL assay is based on a presence of nicks in the DNA which can be identified by terminal deoxynucleotidyl transferase (TdT) at 3' OH ends, ad the 3' OH ends bind to dUTPs that are secondarily labeled with a fluorescent marker. After a coloration, the cells suffered the apoptosis can be identified.

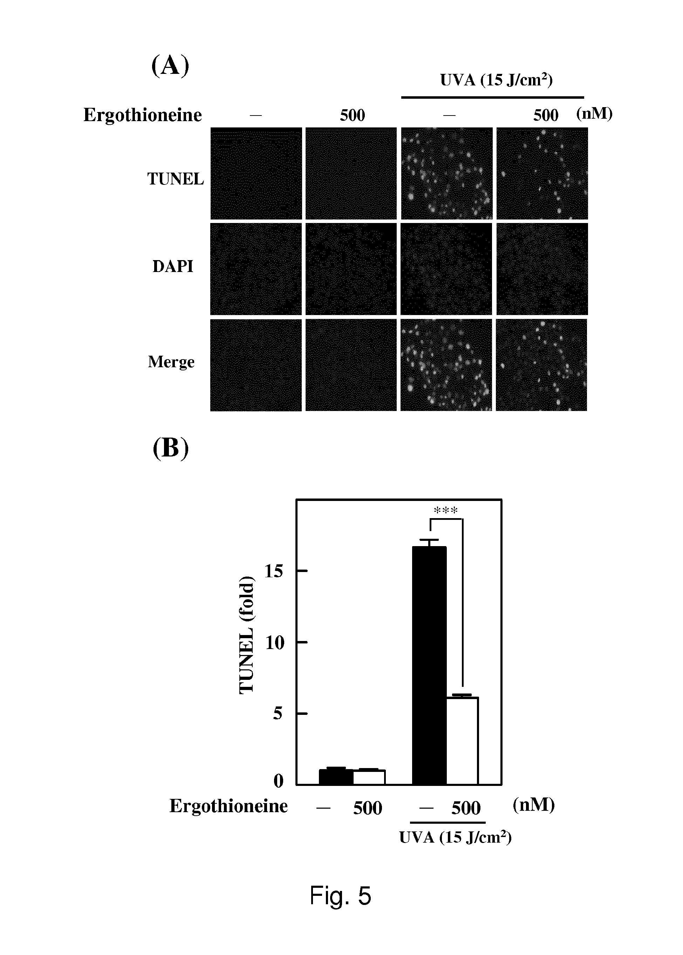

The HaCaT cells are incubated with or without the ergothioneine for 24 hours, wherein the concentration of the ergothioneine is 500 nM. After incubation, the HaCaT cells are exposed to the UVA (15 J/cm²), and then cultured for 4 hours. Then, the HaCaT cells are incubated with TUNEL reaction buffer. The stained cells are visualized using the fluorescence microscope and photographed. Fig. 5 illustrates the effect of the ergothioneine on the apoptosis of the UVA-irradiated HaCaT cells. Fig. 5-(A) is the fluorescent photomicrograph of the TUNEL assay (200 × magnification). Fig. 5-(B) is the quantitative diagram of the TUNEL assay. The data result is represented by mean ± SD values; n = 3, wherein * represents p<0.05 compared to the control group. In Fig. 5, the group treated with the UVA irradiation alone produces a significantly amount of fluorescence (compared with the control group). However, the amount of fluorescence of the group treated the ergothioneine prior to the UVA irradiation is enormously reduced (compared with the group treated with the UVA irradiation alone).

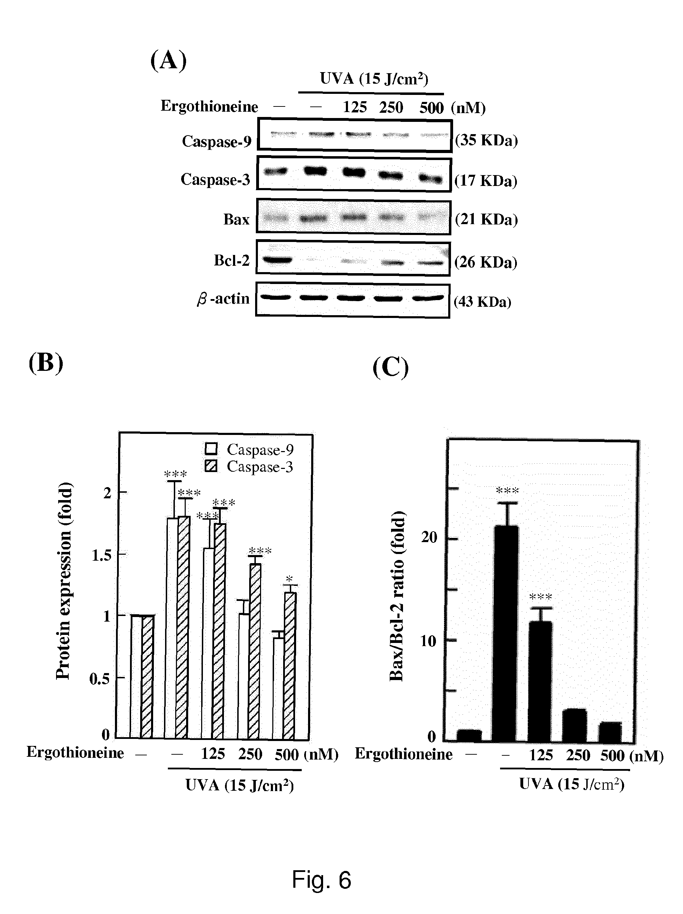

In addition, the HaCaT cells are incubated with or without the ergothioneine for 24 hours, wherein the concentration of the ergothioneine is 125 nM, 250 nM and 500 nM, respectively. After incubation, the HaCaT cells are exposed to the UVA (15 J/cm²), and then incubated for 4 hours. Then, the protein expressions of Caspase-3, Caspase-9, Bax and Bcl-2 are measured using the Western blot analysis. Fig. 6 illustrates the effect of the ergothioneine on the protein expressions of apoptosis-related proteins of the UVA-irradiated HaCaT cells. Fig. 6-(A) represents the Western blot analysis results. Fig. 6-(B) is the quantitative diagram of the protein expressions of Caspase-3 and Caspase-9. Fig. 6-(C) is the quantitative diagram of a Bax/Bcl-2 ratio. The data result is represented by mean ± SD values; n = 3, wherein * represents p<0.05 compared to the control group. In Fig.6, the protein expressions of Caspase-3 and Caspase-9 and the Bax/Bcl-2 ratio are remarkably increased in the group exposed to 15 J/cm² UVA alone (compared with the group untreated with the UVA irradiation). However, the protein expressions of Caspase-3 and Caspase-9 and the Bax/Bcl-2 ratio are significantly reduced in the group treated the ergothioneine prior to the UVA irradiation in the dose-dependent manner. It indicates that the ergothioneine pretreatment to the HaCaT cells can reduce the UVA-induced apoptosis.

### II. The ergothioneine activates an Nrf2 signaling pathway of a damaged cell

### 2.1 The effect of the ergothioneine on expressions of Nrf2 pathway associated proteins of the human keratinocytes

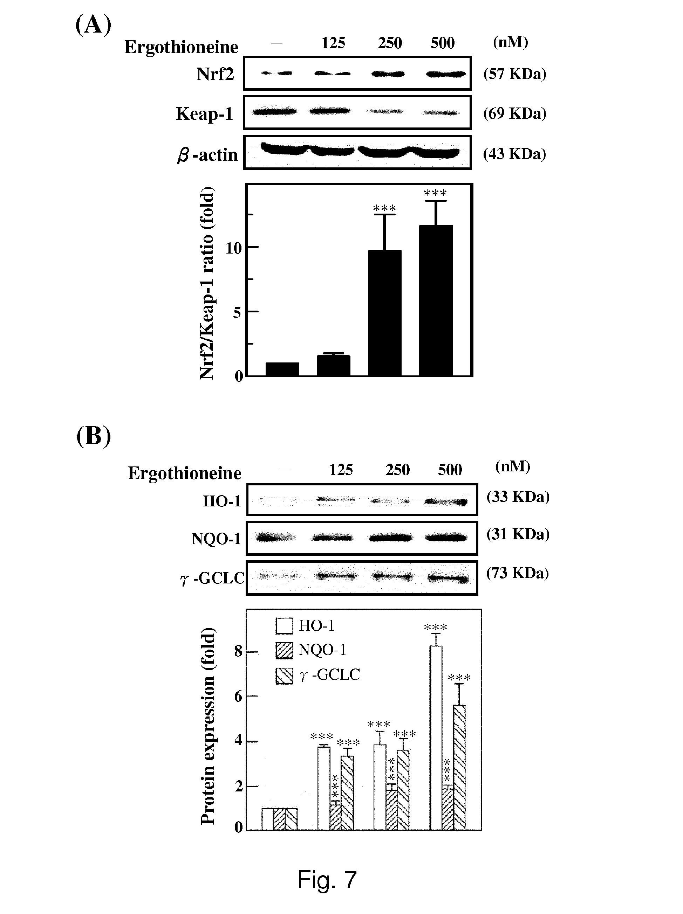

The effect of the ergothioneine on expressions of Nrf2 protein and downstream proteins of the HaCaT cells is monitored by the Western blot analysis. The detected downstream proteins are heme oxygenase-1 (HO-1), γ-glutamylcysteine ligase catalytic subunit (γ-GCLC) and NAD(P)H: quinone acceptor oxidoreductase 1 (NQO-1). The HaCaT cells are incubated with or without the ergothioneine for 1 hour or 4 hours, wherein the concentration of the ergothioneine is 125 nM, 250 nM and 500 nM, respectively. After incubation, the HaCaT cells are suspended in a lysis buffer to collect the proteins. Fig. 7 illustrates the effect of the ergothioneine on the expressions of the Nrf2 signaling pathway associated proteins of the HaCaT cells. Fig. 7-(A) represents the Western blot analysis results of total Nrf2 and Keap-1 protein levels, wherein the HaCaT cells are treated with the ergothioneine (125-500 nM) for 1 hour. Fig. 7-(B) represents the Western blot analysis results of the HO-1, the NQO-1 and the γ-GCLC proteins, wherein the HaCaT cells are treated with the ergothioneine (125-500 nM) for 4 hour. The data result is represented by mean ± SD values; n = 3, wherein * represents p<0.05 compared to the control group. The Western blot analysis results demonstrated that the ergothioneine treatment for 1 hour increases the total Nrf2 protein level, especially with 500 nM ergothioneine. Furthermore, the expressions of the HO-1, the NQO-1 and the γ-GCLC proteins, which are regulated by Nrf2 transcription factor, are also increased. These data elucidate the possible involvement of a Nrf2 transcription factor activation behind the induction of the Nrf2 signaling pathway.

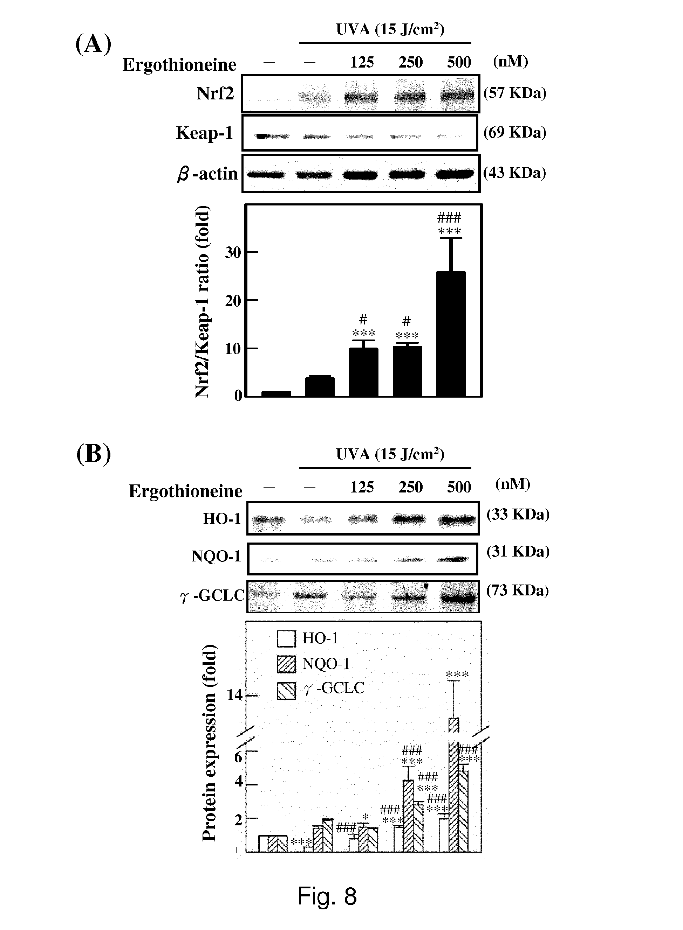

In addition, we examine the effect of the ergothioneine on the expressions of the Nrf2 and the Nrf2 pathway associated proteins of the HaCaT cells treated with the UVA irradiation. The HaCaT cells are incubated with or without the ergothioneine for 24 hours, wherein the concentration of the ergothioneine is 125 nM, 250 nM and 500 nM, respectively. After incubation, the HaCaT cells are exposed to the UVA (15 J/cm²), and then cultured for 1 hour or 4 hours. Next, the HaCaT cells are suspended in the lysis buffer to collect the proteins, and the protein expressions are measured by the Western blot analysis. Fig. 8 illustrates the effect of the ergothioneine on the expressions of the Nrf2 signaling pathway associated proteins of the UVA-irradiated HaCaT cells. Fig. 8-(A) represents the Western blot analysis results of total Nrf2 and Keap-1 protein levels, wherein the HaCaT cells are pretreated with the ergothioneine (125-500 nM), and then exposed to the UVA for 1 hour. Fig. 8-(B) represents the Western blot analysis results of the HO-1, the NQO-1 and the γ-GCLC proteins, wherein the HaCaT cells are pretreated with the ergothioneine (125-500 nM), and then exposed to the UVA for 4 hour. The data result is represented by mean ± SD values; n = 3, wherein * represents p<0.05 compared to the control group, and # represents p<0.05 compared to the group exposed to the UVA. In Fig. 8, the UVA irradiation slightly increases the total Nrf2 expression, and the ergothioneine pretreatment dramatically increases the Nrf2 expression, as seen in the Nrf2/Keap-1 ratio. Furthermore, the expressions of the HO-1, the NQO-1 and the γ-GCLC proteins are also increased in the group pretreated with the ergothioneine.

### 2.2 The ergothioneine promotes an Nrf2 nuclear translocation

The results of the example 2-1 are demonstrated that the ergothioneine can activate the expression of the Nrf2. This example next investigates the effect of the ergothioneine on the Nrf2 nuclear translocation. The HaCat cells are pretreated with the ergothioneine for 24 hours, and then exposed to 15 J/cm² UVA for 1 hour. The protein expressions of a cytosolic Nrf2 and a nuclear Nrf2 are detected by the Western blot analysis, and a nuclear import of Nrf2 of the HaCaT cell is monitored by an immunofluorescence staining.

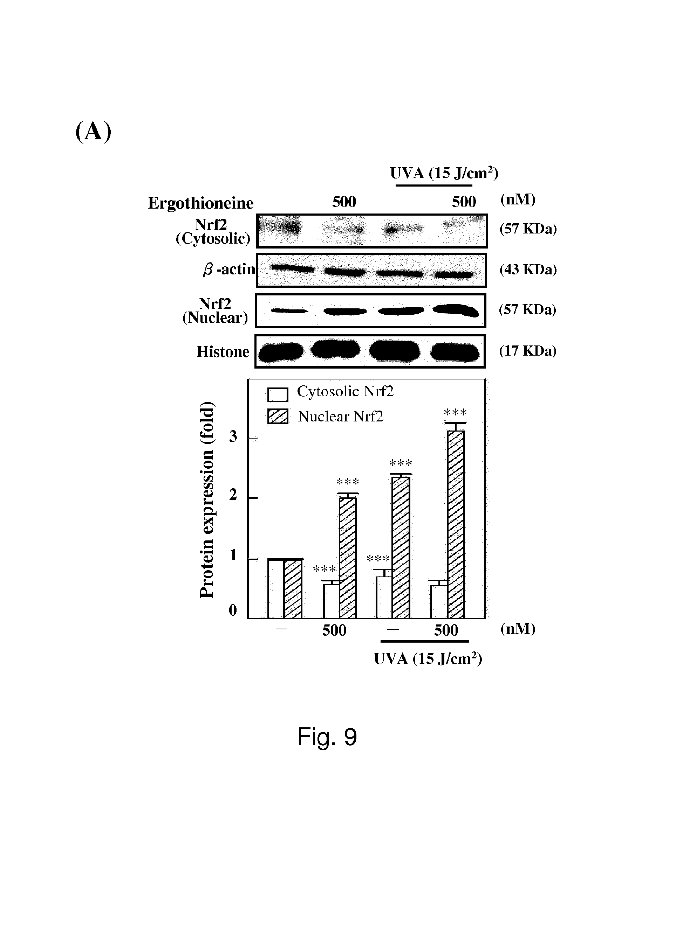

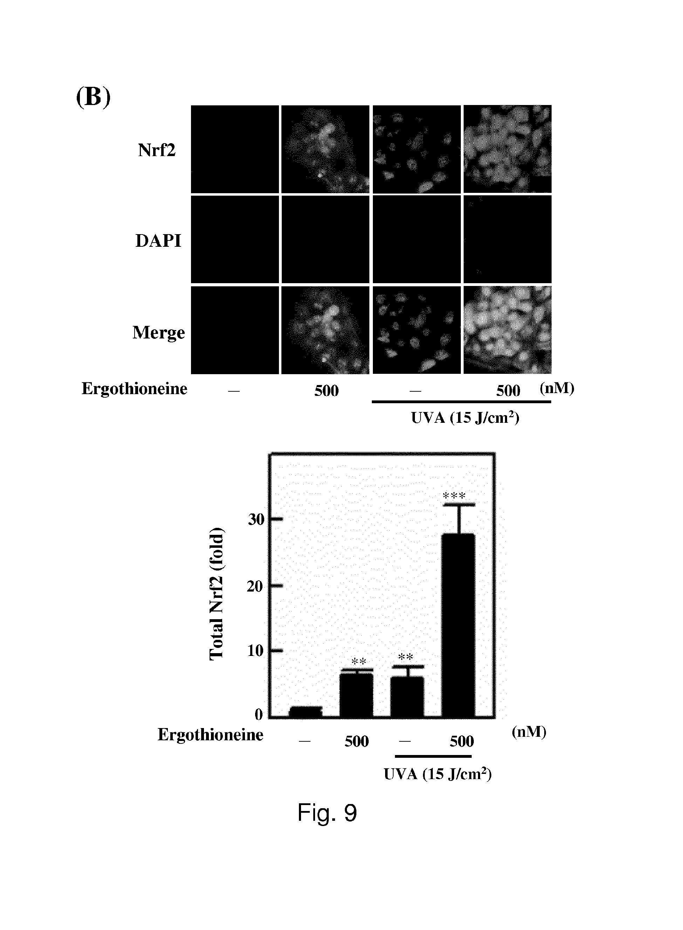

Fig. 9 illustrates the effect of the ergothioneine on the protein expressions of the cytosolic Nrf2 and the nuclear Nrf2 of the UVA-irradiated HaCaT cells. Fig. 9-(A) represents the Western blot analysis result. Fig. 9-(B) is the fluorescent photomicrograph of the immunofluorescence staining (200 × magnifications). The data result is represented by mean ± SD values; n = 3, wherein * represents *p*<0.05 compared to the control group. In Fig. 9-(A), the nuclear Nrf2 protein expression of the group treated the ergothioneine alone is slightly increased (compared to the control group). The nuclear Nrf2 protein expression of the group treated the UVA irradiation alone is also increased (compared to the control group). The nuclear Nrf2 protein expression of the group treated with the ergothioneine prior to the UVA irradiation is significantly increased, and the increased nuclear Nrf2 protein expression of this group is more than the increased nuclear Nrf2 protein expression of the group treated with the ergothioneine alone and the group treated with the UVA irradiation alone. Furthermore, the cytosolic Nrf2 protein expression of the experiment group (the group treated with the ergothioneine alone, treated with the UVA irradiation alone, or treated with the ergothioneine prior to the UVA irradiation) is significantly decreased compared to the control group. It indicates that the ergothioneine treatment can promote the Nrf2 from the cytoplasm enter the nucleus to activate the Nrf2 signaling pathway. In Fig.9-(B), the nucleus is labelled with 4',6-diamidino-2-phenylindole (DAPI), and the Nrf2 is labelled with green fluorescence to measure the expression and a localization of the Nrf2. The immunofluorescence staining result shows that a fluorescence intensity of the Nrf2 is slightly increased and simultaneously a small amount of the Nrf2 enters the nucleus in the group treated with the ergothioneine alone. The nuclear Nrf2 is also spontaneously increased after the UVA irradiation alone. Moreover, the pronounced fluorescence intensity of the nuclear Nrf2 is observed in the group treated with the ergothioneine prior to the UVA irradiation according to the fluorescent photomicrograph. These results indicate that an external stress-stimulated cell promotes the small amount of the Nrf2 entering nucleus by its protection mechanism. Moreover, the ergothioneine promotes enormous Nrf2 from the cytoplasm entering the nucleus after stimuli.

### 2.3 The effect of the ergothioneine on an UVA-induced oxidative damage

A glutathione (GSH), the antioxidant in animal cells, can protect DNA against external oxidative stresses. Given that the ergothioneine treatment to the HaCaT cells can reduce the DNA damage of the HaCaT cells; this example next investigates the UVA-induced oxidative damage by determining a total GSH content of the HaCaT cells treated with the ergothioneine or the UVA irradiation.

A glutathione disulfide (GSSG) is an oxidized form of the GSH, and the GSSG can be reduced to the GSH by a glutathione reductase. Antioxidant enzymes, such as glutathione peroxidases and peroxiredoxins, generate glutathione disulfide during the reduction of peroxides such as hydrogen peroxide (H₂O₂) and organic hydroperoxides (ROOH). The GSH generates a yellow-colored 5-thio-2-nitrobenzoic acid (TNB) and a yellow-colored GSTNB (between GSH and TNB) during the reaction of 5,5'-dithiobis-(2-nitrobenzoic acid) (DTNB), wherein the content of (GSSG+GSH) is the rate determining factor of color production in aforementioned reactions. The rate of the yellow-colored TNB production is directly proportional to the concentration of (GSSG+GSH) in a sample. Thus, measurement of the absorbance of the TNB provides an accurate estimation of the amount of total glutathione (GSSG+GSH) in the sample.

The HaCaT cells are seeded in a 10-cm dish with 1×10⁶ cells/dish and then maintained at 37°C in the humidified atmosphere of 5% CO₂ untill the HaCaT cells are adhered in the next day. The HaCaT cells are subsequently incubated with or without the ergothioneine, and then maintained at 37°C in the humidified atmosphere of 5% CO₂ for 24 hours, wherein the concentration of the ergothioneine is 125 nM, 250 nM and 500 nM, respectively. Next, the HaCaT cells are washed with PBS three times and then irradiated with 15 J/cm² UVA. After the irradiation, the PBS is replaced with the culture medium to culture the HaCaT cells for 4 hours. In next step, the HaCaT cells are collected in a 1.5 mL eppendorf and resuspended in 1 mL of MES buffer. The suspended HaCaT cells are lysed by a sonicator. Then the cell lysis is centrifuged at 10,000 × g in 4°C for 5 minutes, and then the supernatant is collected. 50 µL of the supernatant or 50 µl of a standard solution is added into a 96-well microplate, and then 150 µL of an Assay Cocktail is added in each well to act for 25 minutes in the dark, wherein the Assay Cocktail is prepared in advance as following proportions: 8.156 mL of MES buffer, 0.326 mL of cofactor mixture, 1.522 mL of enzyme mixture, 1.667 mL of ddH₂O, and 0.326 mL of DTNB (a total volume of liquid is 12 mL). The net absorbance at 405 nm yields an accurate amount of the total glutathione in the sample.

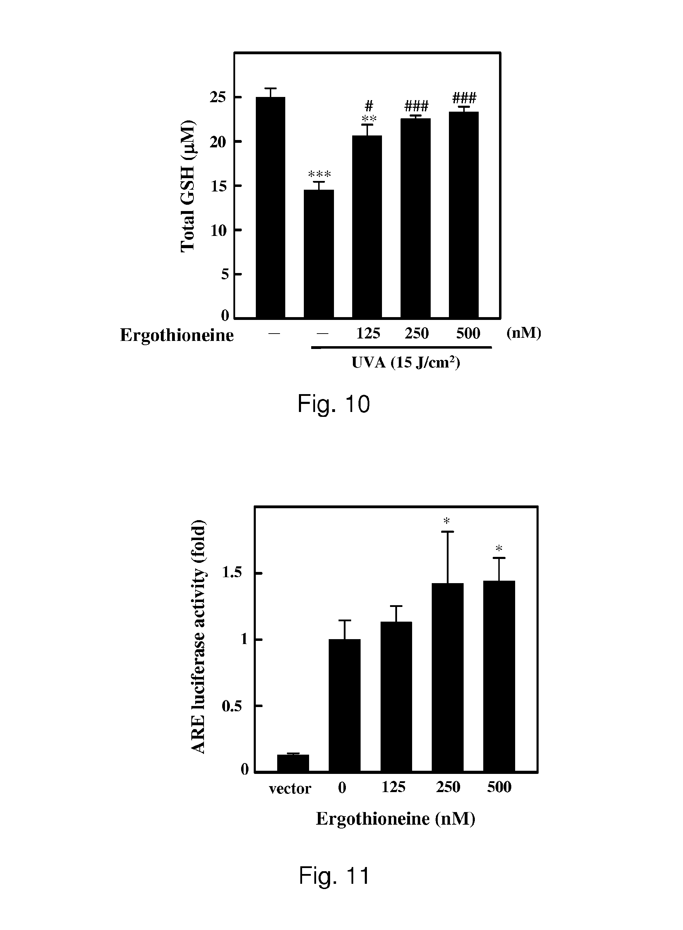

Fig. 10 illustrates the effect of the ergothioneine on a content of a glutathione (GSH) of the UVA-irradiated HaCaT cells. The data result is represented by mean ± SD values; n = 3, wherein * represents p<0.05 compared to the control group, and # represents p<0.05 compared to the group exposed to the UVA. The UVA irradiation remarkably depleted the total glutathione levels compared to the control group. Interestingly, the total glutathione level is increased in the group treated with the ergothioneine prior to the UVA irradiation in the dose-dependent manner. It indicates that the ergothioneine has the protective properties in maintaining the intracellular total GSH content and reducing the oxidative damage.

### 2.4 The effect of the ergothioneine on transcriptions of antioxidant enzymes

The Nrf2 binds to the small Maf after translocating into the nucleus. The Nrf2-sMaf heterodimer then binds to the ARE to enhance the transcriptional activity of the promoter, hence the Nrf2-Keap-1 signaling pathway is activated. Given that the ergothioneine treatment to the HaCaT cells can promote the Nrf2 translocating into the nucleus; this example next investigates whether the ARE transcriptional activity can be started by using a luciferase gene reporter assay. The luciferase gene reporter assay is transfected a plasmid containing the ARE gene and a luciferase gene into the HaCaT cells, wherein the luciferase gene is a reporter gene. An internal standard of the luciferase gene reporter assay is β-galactosidase, and a luminescence intensity is measured by Dual Light Kit (Applied Biosystems).

Fig. 11 illustrates the effect of the ergothioneine on the transcriptional activity of the ARE. The data result is represented by mean ± SD values; n = 3, wherein * represents p<0.05 compared to the control group. The luciferase activity derived from the ARE promoter is increased in the HaCaT cells treated with the ergothioneine for 4 hours (compared to the HaCaT cells untreated with the ergothioneine). It indicates that the ergothioneine can enhance the transcriptional activity of the ARE promoter.

### 2.5 Discussion of an ergothioneine-induced Nrf2 signaling pathway activation

Given that the ergothioneine can activate the Nrf2 to induce the expression of the downstream antioxidant genes; this example further investigates the pathway of the Nrf2 activated by the ergothioneine. Previously studies have demonstrated that the Nrf2 is phosphorylated by a mitogen-activated protein kinases (MAPK), a phosphoinositide 3-kinase (PI₃K) and a protein kinase C (PKC) pathway to separate the Nrf2 and the Keap-1, and then the Nrf2 translocates into the nucleus.

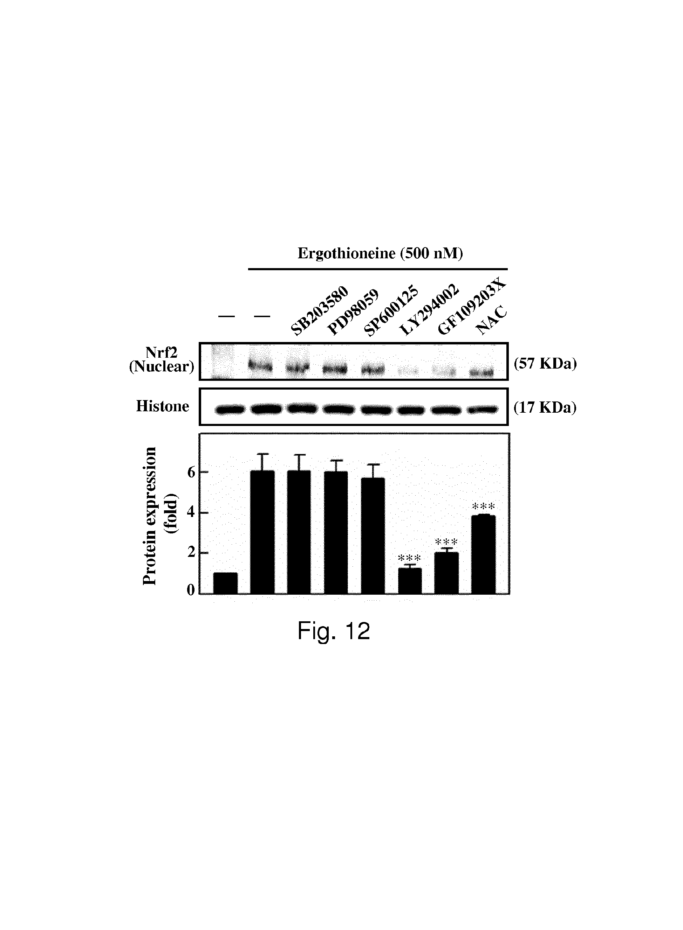

The HaCaT cells are incubated with respective pharmacological inhibitors of p38 (SB203580; 20 µM), ERK (PD98059; 20 µM), JNK (SP600125; 20 µM), PI₃K (LY294002; 20 µM), PKC (GF109203X; 2.5 µM), or ROS (NAC; 1 mM) for 30 minutes, and then incubated with 500 nM ergothioneine for 1 hour. After the incubation, nuclear extracts and cytosolic extracts are collected to observe the expression of the Nrf2 nuclear translocation. Fig. 12 illustrates that the ergothioneine induces a Nrf2-mediated antioxidant signaling pathway. The data result is represented by mean ± SD values; n = 3, wherein * represents p<0.05 compared to the control group. The Western blot analysis result indicates that the expression of the Nrf2 nuclear translocation is significantly inhibited by the PI₃K inhibitor (LY294002) and the PKC inhibitor (GF109203X).

### 2.6 Discussion of a relationship between a protective effect of the ergothioneine and between the Nrf2

The Nrf2 is an important transcription factor, the downstream antioxidant enzymes are regulated by the Nrf2 transcription gene. To examine protective effects of the ergothioneine on the HaCaT cells indeed through the Nrf2 activation, we developed an Nrf2 gene knockdown model in the HaCaT cells by a siRNA transfection in this example. Then, the expression of the downstream antioxidant gene is observed to investigate whether the protective properties of the ergothioneine will be affected.

The siRNA transfection includes steps as follows. The HaCaT cells are seeded in a 6-well plate with 4×10⁵ cells/well, and then maintained at 37°C in the humidified atmosphere of 5% CO₂ untill the time of the transfection. The day after reaching 40-60% confluence, the culture medium is replaced with 1 mL of FBS-free and antibiotic-free culture medium in each well, and the HaCaT cells are transfected with a siNrf2 or a control siRNA by using Lipofectamine 2000 (Invitrogen, U.S.A.) for 4-6 hours. After the incubation for 4-6 hours, the HaCaT cells are washed with the culture medium supplemented with 10% FBS 2-3 times, and then cultured in the culture medium supplemented with 10% FBS for 12-16 hours.

After the siRNA transfection, the HaCaT cells are treated with 500 nM ergothioneine for 24 hours. After the ergothioneine treatment, the HaCaT cells are subjected to analyses of the protein expression of the Nrf2 in 1 hour post-treatment and the protein expressions of HO-1, γ-GCLC and NQO-1 in 4 hours post-treatment by the Western blot analysis. Fig. 13-(A) illustrates the effect of the ergothioneine on protein expression of the Nrf2 in Nrf2 knockdown HaCaT cells by using the siRNA transfection. In Fig. 13-(A), successful knockdown of the Nrf2 is confirmed by the Western blot analysis, as indicated by a disappearance of the Nrf2 protein band in the HaCaT cell transfected with the Nrf2 siRNA. In addition, the HaCaT cells transfected with the Nrf2 siRNA are not shown the increased expression of HO-1, NQO-1 and γ-GCLC with the ergothioneine treatment. It indicates that the HO-1, NQO-1 and γ-GCLC antioxidant gene are not activated.

We further examine whether the ergothioneine is capable of inhibiting the photooxidation through the Nrf2 activation. The HaCaT cells are incubated with 500 nM ergothioneine for 24 hours after the siRNA transfection. After the incubation, the HaCaT cells are treated with or without the UVA irradiation (15 J/cm²) for 4 hours. Then the cell viability of the HaCaT cells is measured by the MTT assay to observe whether the ergothioneine still has the protective property in Nrf2 knockdown cells. Fig. 13-(B) illustrates the effect of the ergothioneine on the cell viability in Nrf2 knockdown HaCaT cells by using the siRNA transfection. The data result is represented by mean ± SD values; n = 3, wherein * represents *p*<0.05 compared to the control group. In Fig. 13-(B), the ergothioneine treatment is able to reduce a UVA-induced cell death in the group transfected with the control siRNA. However, the ergothioneine pretreatment is unable to ameliorate the UVA-induced cell death in the group transfected with the Nrf2 siRNA.

We further measure the amount of ROS generation in the HaCaT cells treated with the ergothioneine prior to 15 J/cm² UVA irradiation. The HaCaT cells are incubated with 500 nM ergothioneine for 24 hours after the siRNA transfection. After the incubation, the HaCaT cells are treated with or without the UVA irradiation (15 J/cm²) for 4 hours, and then the HaCaT cells are subjected to detect the ROS generation. Fig. 13-(C) illustrates the effect of the ergothioneine on the ROS generation of the UVA-irradiated HaCaT cells by using the siRNA transfection. The data result is represented by mean ± SD values; n = 3, wherein * represents p<0.05 compared to the control group. In the groups transfected with the control siRNA, the ROS generation of the group treated with the ergothioneine prior to the UVA irradiation is significantly reduced compared to the group treated with the UVA irradiation alone. However, the ergothioneine pretreatment is unable to decrease the ROS generation in the groups transfected with the Nrf2 siRNA. It suggests that the property of the ergothioneine in reducing the UVA-induced ROS generation is regulated by the activation of Nrf2 signaling pathway in the HaCaT cells.

Finally, we further examine the protective property of the ergothioneine on the inhibition of the apoptosis in the HaCaT cells exposed to the UVA using the TUNEL assay. Fig. 13-(D) illustrates the effect of the ergothioneine on the apoptosis of the UVA-irradiated HaCaT cells by using the siRNA transfection. The data result is represented by mean ± SD values; n = 3, wherein * represents p<0.05 compared to the control group. In the groups transfected with the control siRNA, the apoptosis of the group treated with the ergothioneine prior to the UVA irradiation is significantly decreased compared to the group treated with the UVA irradiation alone. In contrast, the ergothioneine pretreatment is unable to improve the UVA-induced apoptosis in the groups transfected with the Nrf2 siRNA. It indicates that the property of the ergothioneine in inhibiting the UVA-induced apoptosis is regulated by the activation of Nrf2 signaling pathway in the HaCaT cells.

To sum up, the present disclosure provides the use of the ergothioneine; the ergothioneine can activate the Nrf2 signaling pathway of the human keratinocytes and promote the Nrf2 nuclear translocation to enhance the expression of the downstream enzymatic antioxidant and non-enzymatic antioxidant. Thus, the ergothioneine can increase the anti-photooxidation ability of the human keratinocytes. Therefore, the ergothioneine of the present disclosure can be used to prepare pharmaceutical compositions to induce the activity of the Nrf2, the pharmaceutical compositions to inhibit the UVA-induced apoptosis, the pharmaceutical compositions to enhance the expression of enzymatic antioxidant or non-enzymatic antioxidant, and the pharmaceutical compositions to promote the Nrf2 nuclear translocation. In addition, a therapeutically effective amount of the ergothioneine only ranges from 125 nM to 500 nM.

## Claims

1. A use of an ergothioneine in manufacture of a product for inducing an activity of an Nrf2 in a cell.

2. The use of the ergothioneine according to claim 1, wherein the cell is a normal cell or a damaged cell.

3. The use of the ergothioneine according to claim 2, wherein the damaged cell results from an exposure to an ultraviolet radiation (UVR).

4. The use of the ergothioneine according to claim 3, wherein the UVR is an ultraviolet A (UVA) with a wavelength from 320 nm to 400 nm.

5. The use of the ergothioneine according to claim 4, wherein the ergothioneine is used in manufacture of the product for inhibiting an apoptosis caused by a stimulation of the UVA.

6. The use of the ergothioneine according to claim 1, wherein the ergothioneine is used in manufacture of the product for increasing the expression of Nrf2-mediated genes expressing an enzymatic antioxidant.

7. The use of the ergothioneine according to claim 6, wherein the enzymatic antioxidant is selected from the group consisting of heme oxygenase-1 (HO-1), NAD(P)H:quinone oxidoreductase 1 (NQO1), and glutamate-cysteine ligase catalytic subunit (GCLC).

8. The use of the ergothioneine according to claim 1, wherein the ergothioneine is used in manufacture of the product for increasing the expression of the Nrf2-mediated genes expressing a non-enzymatic antioxidant.

9. The use of the ergothioneine according to claim 8, wherein the non-enzymatic antioxidant is a glutathione (GSH).

10. The use of the ergothioneine according to claim 1, wherein the ergothioneine is used in manufacture of the product for inducing a nuclear translocation of the Nrf2.

11. The use of the ergothioneine according to claim 1, wherein a therapeutically effective amount of the ergothioneine ranges from 125 nM to 500 nM.
